# EUROPEAN PATENT SPECIFICATION

(11) **EP 1 073 625 B1**
(45) Date of publication and mention of the grant of the patent: **11.06.2003**
(21) Application number: 99916009.6
(22) Date of filing: 09.04.1999
(51) Int. Cl.: C07C 229/48, A61K 31/215

(54) **TILIDINE MESYLATE, PROCESSES FOR ITS PREPARATION AND PHARMACEUTICAL COMPOSITION THEREOF**
TILIDIN-MESYLAT, VERFAHREN ZU DESSEN HERSTELLUNG UND PHARMAZEUTISCHE ZUSAMMENSETZUNGEN
MESYLATE DE TILIDINE, PROCEDES DE PREPARATION ASSOCIES ET COMPOSITION PHARMACEUTIQUE LE CONTENANT

(30) Priority: 28.04.1998 IE 980322
(43) Date of publication of application: 07.02.2001
(73) Proprietor: RUSSINSKY LIMITED, Cork (IE)
(72) Inventor: SHICKANEDER, Helmut, South Terrace, Cork (IE); NIKOLOPOULOS, Aggelos, Cork (IE); BRUTON, Brian, County Cork (IE)
(74) Representative: O'Brien, John Augustine
(86) International application number: IE9900024
(87) International publication number: WO99055662

(56) References cited:
- WO-A-94/10129
- DE-A- 1 923 620
- DE-A- 2 107 871
- DE-B- 1 768 704
- DE-B- 2 132 562
- US-A- 3 557 127
- CHEMICAL ABSTRACTS, vol. 101, no. 23, 3 December 1984 Columbus, Ohio, US; abstract no. 210763f, page 615; column 2; XP002106871 & CS 214 312 A (DAVID, LADISLAV ET AL.)
- CHEMICAL ABSTRACTS, vol. 86, no. 25, 20 June 1977 Columbus, Ohio, US; abstract no. 183113g, DE LA IGLESIA, F. A. ET AL.: "The action of tilidine fumarate on drug-metabolizing enzymes and ultrastructure of rat liver." page 36; column 2; XP002106872 & PROC. EUR. SOC. TOXICOL., vol. 17, 1976, pages 220-233,

## Description

The invention relates to a salt of Tilidine, processes for its preparation and its use in pharmaceutical products.

(±)-Ethyl-*trans*-2-(dimethylamino)-1-phenyl-3-cyclohexene-1-carboxylate (Tilidine) is a pharmaceutically active substance with strong analgesic properties. Trans-Tilidine Base (1) is known to be a compound with a very low melting point [Pharm. Ind. 40(6), 657-664 [1978]; Cienc. Ind. Farm. 8(1), 4-11 [1976]). This creates problems for the manufacture of final pharmaceutical products.

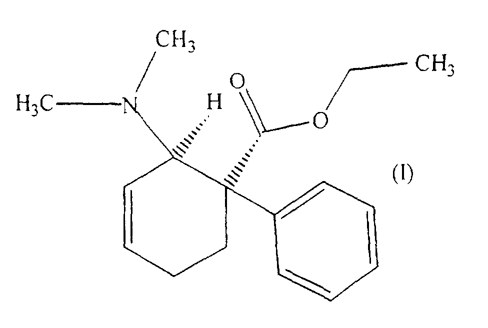

Therefore Tilidine is generally used in pharmaceuticals in the form of the Hydrochloride Semihydrate which is a more stable compound than Tilidine base. However, the Hydrochloride Semihydrate is only suitable for the preparation of solutions and certain types of soft capsules. Hydrochloride Semihydrate is not suitable for "solid formulations" required for the production of tablets and the like.

WO-A-94/10129, describes Tilidine Dihydrogen Orthophosphate, which is said to be stable and therefore suitable for the preparation of solid pharmaceutical formulations such as tablets or dragees. However the process for the preparation of Tilidine Dihydrogen Orthophosphate appears to be critical and complicated.

There is therefore a need for a new chemical entity based on Tilidine which is stable and can be easily produced in high yield and good quality.

This invention is directed towards providing such a new entity.

More particularly the object of the present invention is to provide a novel entity based on Tilidine which is easy to produce, which shows high stability, an improved taste and a suitable pH range in aqueous solutions and which is useful for liquid and especially solid formulations, particularly with a retard profile.

### Statements of Invention

The invention provides Tilidine Mesylate.

The invention also provides a process for the preparation of Tilidine Mesylate comprising:-
reacting Tilidine base with Mesylic Acid in an appropriate solvent; and
isolating the desired salt.

In one embodiment of the invention the solvent is ethyl acetate.

In another embodiment of the invention the solvent is acetone.

In another embodiment of the invention the solvent is MIBK.

Preferably the reaction temperature is from 0° to 80°C, most preferably from 0° to 40°C.

The invention further provides a pharmaceutical composition containing Tilidine Mesylate as an active ingredient.

The composition may be in a liquid or solid form. Preferably the composition is in the form of a tablet, dragee, capsule, suppository or granulate.

The composition may be in a retard formulation for sustained release.

The invention will be more clearly understood from the following description given by way of example only.

### Description of the Invention

It has been found that Tilidine Mesylate can be obtained via a simple process. The salt is very stable, shows attractive solubility and pH properties and is suitable for solid and liquid formulations.

According to the literature (WO-A-94/10129) Tilidine salts are difficult to crystallise. Surprisingly therefore we have found that Tilidine Mesylate can be crystallised as a 1:1-adduct by reacting Tilidine base and Mesylic Acid in various solvents such as Ethyl Acetate. The product can be isolated as a white to off-white, odourless powder in very high yields. The salt shows a pH value in the range of 4 - 5, approximately 4.5 (1%-5% aqueous solution). It was found that the Mesylate is very stable over a long period of time as a solid and in solution. Furthermore the Mesylate is characterised by an improved taste compared to the Hydrochloride Semihydrate and is non-hygroscopic in contrast to Tilidine Hydrochloride Semihydrate and Dihydrogen Orthophosphate.
As Tilidine Mesylate is very stable, and has a high melting point (136°C) it is very suitable for the manufacture of granulates which may be converted into tablets, dragees or pills. A delayed action of the active compound Tilidine may therefore be obtained.

In preparing such compositions there can be employed any of the various adjuvant or excipient materials customarily employed in the art. These adjuvants or excipients include for example inert substances such as water, gelatine, lactose, vegetable oil as well as many of the other compatible materials conventionally used in the preparation of medicaments.

As Tilidine Mesylate also shows excellent solubility in water giving almost neutral and stable solutions the Mesylate has significantly improved properties compared to the Hydrochloride Semihydrate and the Dihydrogen Orthophosphate.

The process for the preparation of Tilidine Mesylate is extremely simple and efficient. The product is isolated in a relatively simple procedure at high yields, greater then 75% and at a very high level of purity, greater than 99%.

Tilidine Mesylate may be administered in suitable quantities, depending on the amount of Tilidine to be provided. Typically Tilidine Hydrochloride is administered in doses of up to 400mg/ day, usually in 50 mg units in the form of capsules or drops. To achieve a similar profile an appropriate single dose of Tilidine Mesylate is approximately 60mg and the maximum amount administered per day is 450 to 500 mg. In the case of retard formulations, preferably Naloxon Hydrochloride Dihydrate is used as an opiate antagonist in the formulation.

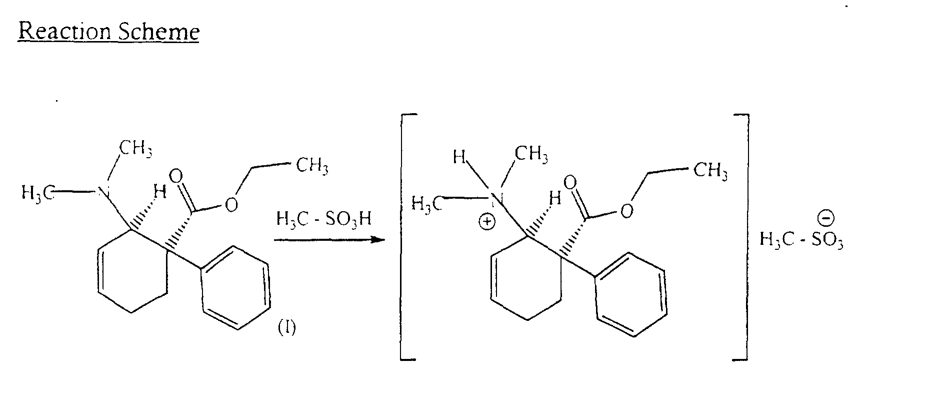

### Example 1

### Preparation of Tilidine Mesylate

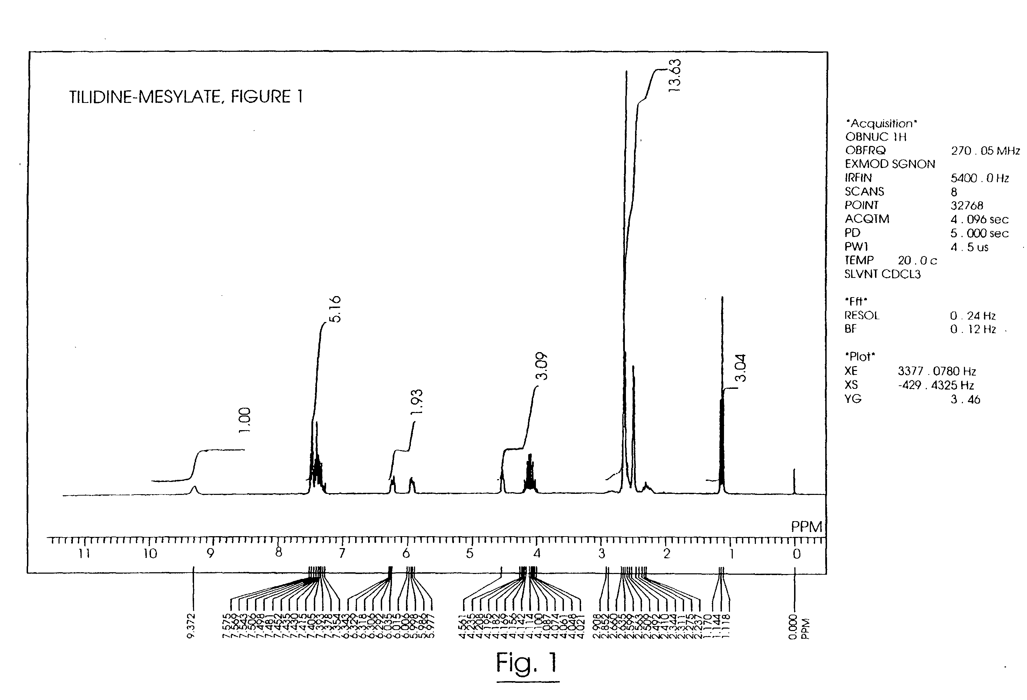

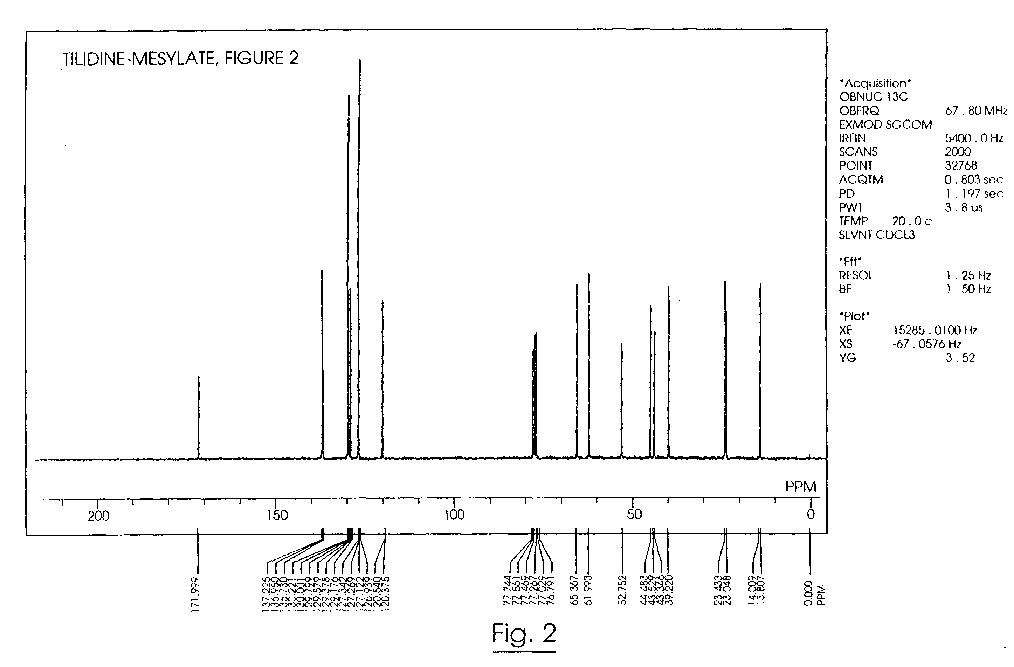

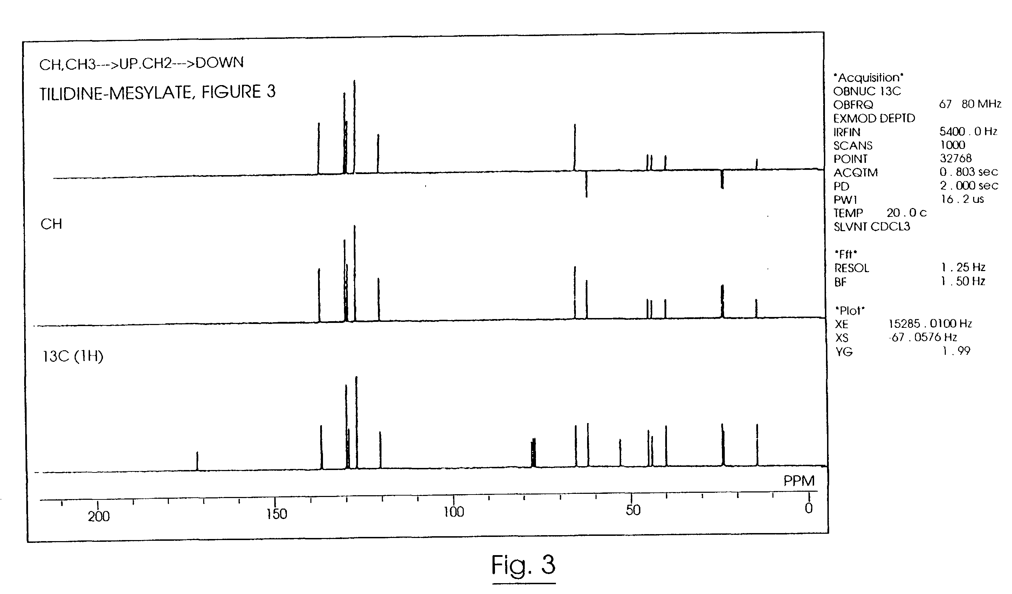

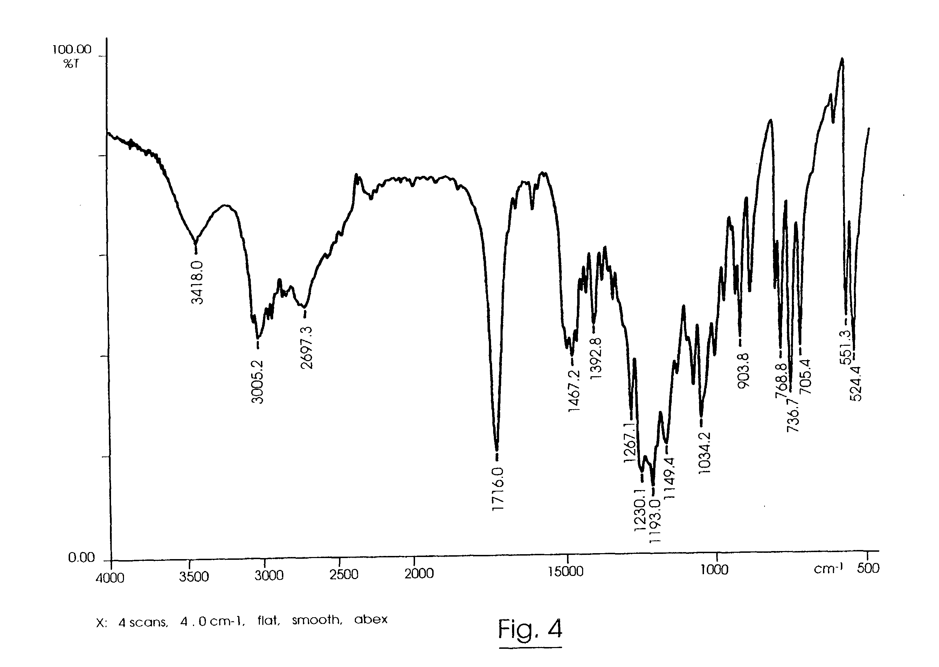

30 g (0.01 mol) of Tilidine Base were dissolved in 50 ml of Ethyl Acetate at 0°-80°C, preferably 0°- 40°C, and 10.5 g (0.01 mol) of Mesylic Acid were added. The mixture was cooled to room temperature to precipitate the product as a white to off-white salt. The product was filtered off and washed with Ethyl Acetate and was dried under vacuum. A typical yield of 38.1g or approximately 99% was obtained with a melting point of 136°C. The following are shown in figures 1, 2, 3 and 4 respectively; ¹H-NMR-spectrum, ¹³C-NMR-spectrum, DEPT and the IR-spectrum.

Microanalysis of the compound revealed the following:
calculated: C58.51, H 7.37, N 3.79, S 8.68;
found: C 58.65, H 7.46, N3.90, S 8.54.

### pH Values

1% aqueous solution ∼pH 4.7
5% aqueous solution ∼pH 4.4

### Stability

Tilidine Mesylate was stored as a solid over a long period of time under standard conditions. No critical change in purity became evident and no significant degradation of the material was observed.

Tilidine Mesylate was also observed in aqueous solutions at pH values in the range of approximately pH 7.0 to pH1.0. Tilidine Mesylate was found to be exceptionally stable even in the neutral range and no notable decomposition was observed. In contrast Tilidine hydrochloride solutions have to be kept in the acidic range around pH 2.0 to avoid degradation of the material.

### Taste

Organoleptic tests with human volunteers found that Tilidine Mesylate has a less bitter taste than the Hydrochloride Hemihydrate salt.

### Example 2

30g (0.01 mol ) of Tilidine base were dissolved in 50- 100 ml of MIBK at 0°-80° C, preferably 0°- 40° C and 10.5g (0.01 mol) of Mesylic Acid were added. The mixture was stirred at room temperature to allow the product to precipitate. The product was then filtered off and washed with MIBK and dried under vacuum. A yield of 30.4g, approximately 79%, was obtained of Tilidine Mesylate with the characteristics of Example 1.

### Example 3

75 g (0.025 mol) of Tilidine Base were dissolved in 100-300 ml of Acetone at 0°-50°C and 26.3 g of Mesylic Acid were added. The mixture was stirred at room temperature to allow the product to precipitate. The volume was reduced by evaporating part of the solvent. The product was filtered off and washed with Ethyl Acetate and dried under vacuum. A yield of 82 g, approximately 81% of Tilidine Mesylate with the characteristics of Example 1 was obtained.

### Example 4

### Retard formulation containing Tilidine Mesylate

960g Tilidine Mesylate prepared as in example in 1 were added to 70.4g Naloxon Hydrochloride Dihydrate, 740g lactose and 700g hydrated castor oil, slowly stirred and heated up to a temperature of preferably 50-85°C. The mixture was filtered through a filter with a pore size of 2.5mm. After cooling to room temperature, the mixture was further filtered through a filter with a pore size of 1mm. 273.6g of ammonia methyl acrylate co-polymer, 32g Magnesium stearate and 24g silicon dioxide were added to the mixture. The resultant mixture was then compressed into tablet form.

The invention is not limited to the embodiments hereinbefore described which may be varied in detail.

## Claims

1. Tilidine Mesylate.

2. A process for the preparation of Tilidine Mesylate comprising:-
reacting Tilidine Base with Mesylic Acid in an appropriate solvent; and
isolating the desired salt.

3. A process as claimed in claim 2 wherein the solvent is Ethyl Acetate.

4. A process as claimed in claim 2 wherein the solvent is MIBK.

5. A process as claimed in claim 2 wherein the solvent is Acetone.

6. A process as claimed in any of claims 2 to 5 wherein the reaction temperature is from 0° to 80°C preferably from 0° to 40°C.

7. A pharmaceutical composition containing Tilidine Mesylate as an active ingredient.

8. A composition as claimed in claim 7 in a liquid form, in a solid form and/or in the form of a tablet, dragee, capsule, suppository or granulate.

9. A composition as claimed in claim 7 or 8 wherein the composition is in a retard formulation for sustained release.

## Patentansprüche

1. Tilidinmesylat.

2. Verfahren zur Herstellung von Tilidinmesylat umfassend:
Umsetzen von Tilidinbase mit Mesylsäure in einem geeigneten Lösungsmittel; und
Isolieren des gewünschtes Salzes.

3. Verfahren nach Anspruch 2, wobei das Lösungsmittel Ethylacetat ist.

4. Verfahren nach Anspruch 2, wobei das Lösungsmittel MIBK ist.

5. Verfahren nach Anspruch 2, wobei das Lösungsmittel Aceton ist.

6. Verfahren nach einem der Ansprüche 2 bis 5, wobei die Reaktionstemperatur 0° bis 80°C, bevorzugt 0° bis 40°C, beträgt.

7. Pharmazeutische Zusammensetzung, die Tilidinmesylat als aktiven Bestandteil enthält.

8. Zusammensetzung nach Anspruch 7 in flüssiger Form, in fester Form und/oder in Form einer Tablette, eines Dragees, einer Kapsel, eines Zäpfchens oder eines Granulats.

9. Zusammensetzung nach Anspruch 7 oder 8, wobei die Zusammensetzung in einer Retard-Formulierung mit Langzeitwirkung vorliegt.

## Revendications

1. Mésylate de tilidine.

2. Procédé de préparation de mésylate de tilidine, comprenant :
la réaction de la base de tilidine avec l'acide mésylique dans un solvant approprié ; et
l'isolement du sel désiré.

3. Procédé selon la revendication 2, dans lequel le solvant est l'acétate d'éthyle.

4. Procédé selon la revendication 2, dans lequel le solvant est le MIBK.

5. Procédé selon la revendication 2, dans lequel le solvant est l'acétone.

6. Procédé selon l'une quelconque des revendications 2 à 5, dans lequel la température réactionnelle va de 0° à 80°C, préférablement de 0° à 40°C.

7. Composition pharmaceutique, contenant le mésylate de tilidine comme ingrédient actif.

8. Composition selon la revendication 7, sous forme liquide, sous forme solide et/ou sous forme d'un comprimé, d'une dragée, d'une capsule, d'un suppositoire ou d'un granulé.

9. Composition selon la revendication 7 ou 8, dans laquelle la composition est dans une formulation retard pour une libération prolongée.
